# FASCICULE DE BREVET EUROPEEN

(11) **EP 0 674 900 B1**
(45) Date de publication et mention de la délivrance du brevet: **25.11.1998**
(21) Numéro de dépôt: 95400573.2
(22) Date de dépôt: 15.03.1995
(51) Int. Cl.: A61K 9/70

(54) **Système matriciel transdermique d'administration d'un oestrogène et/ou un progestatif à base d'EVA**
Transdermales Matrixsystem auf EVA Basis zur Verabreichung eines Östrogens und/oder Gestagens
Transdermal matrix system comprising EVA for the administration of an oestrogen and/or progestin

(30) Priorité: 28.03.1994 FR 9403598
(43) Date de publication de la demande: 04.10.1995
(73) Titulaire: LABORATOIRES D'HYGIENE ET DE DIETETIQUE, 75008 Paris (FR)
(72) Inventeur: Teillaud, Eric, F-21240 Talant (FR); Sawaya, Antoine, F-21000 Dijon (FR); Math, Marie-Christine, F-21240 Talant (FR)
(74) Mandataire: Clisci, Serge

(56) Documents cités:
- EP-A- 0 371 496
- EP-A- 0 483 370
- FR-A- 2 612 785
- US-A- 4 060 084

## Description

### DOMAINE DE L'INVENTION

La présente invention a pour objet un nouveau système matriciel auto-adhésif pour l'administration d'un composant oestrogène et/ou d'un composant progestatif par voie percutanée à base de copolymère d'éthylène et d'acétate de vinyle (EVA).

### ART ANTERIEUR

On sait que de nombreux systèmes transdermiques d'administration d'un composant oestrogène seul ou en association avec un composant progestatif ont été étudiés durant la dernière décennie, en particulier pour le traitement des symptômes de la ménopause et de l'ostéoporose dans le cadre de traitements qualifiés de "thérapie de remplacement des hormones déficitaires". Des systèmes matriciels dont la matrice est à base de copolymère d'éthylène et d'acétate de vinyle (EVA) et où les principes actifs sont dispersés ou dissous sont notamment décrits dans la demande de brevet publiée FR-A-2 612 785 et, dans le cas de l'administration d'une combinaison oestrogène-progestatif, dans les demandes de brevet publiées EP-A-0 279 982 et EP-A-0 555 360.

Il est aussi connu de l'homme de l'art que, pour les EVAs comme en général pour tous les polymères utilisés dans les systèmes transdermiques, il est impossible, suivant la nature des composés associés à ces derniers et le type du principe actif utilisé, de prévoir les quantités de principe actif libérées au cours du temps et les rendements de ces systèmes.

Ceci est bien illustré par la lecture des exemples de la demande de brevet publiée FR-A-2 635 979 qui décrit des dispositifs à base d'EVA contenant différents principes actifs et qui montre que les résultats obtenus sont en effet très variables.

En pratique, on considère que seuls des principes actifs proches, c'est-à-dire appartenant à une même famille chimique, dissous dans des formulations identiques présentent généralement des propriétés comparables.

On sait également que les oestrogènes et les progestatifs sont des produits (i) qui sont peu solubles dans les polymères de type EVA, et (ii) qui passent difficilement la barrière cutanée.

Ainsi les quantités libérées de ces principes actifs pour obtenir l'effet thérapeutique recherché sont en général faibles par rapport aux quantités initiales présentes dans les dispositifs transdermiques ce qui a pour conséquence l'obtention de rendements faibles.

### BUTS DE L'INVENTION

Selon l'invention on propose donc la réalisation de systèmes matriciels auto-adhésifs pour l'administration d'oestrogènes et/ou de progestatifs qui n'ont pas les inconvénients précités et qui présentent en plus d'excellents rendements.

Selon un premier aspect de l'invention, on se propose de fournir un système matriciel transdermique à base d'EVA et d'un dérivé cellulosique pour l'administration d'un composant oestrogène et/ou d'un composant progestatif.

Selon un second aspect de l'invention, on se propose de fournir un procédé de préparation d'un tel système matriciel.

### OBJET DE L'INVENTION

Les buts précités sont obtenus grâce à une nouvelle solution technique selon laquelle la matrice du système matriciel, qui contient un composant oestrogène et/ou un composant progestatif, est essentiellement constituée d'EVA, d'un dérivé cellulosique et d'un troisième produit choisi parmi le crotamiton, les 2-pyrrolidones N-substituées de formule I ci-après, les alcools aliphatiques supérieurs en C₁₂-C₂₀ et leurs mélanges.

Plus précisément, on préconise selon l'invention un système matriciel transdermique auto-adhésif pour l'administration par voie percutanée d'une hormone, ledit système, qui comprend un support et une matrice auto-adhésive étant caractérisé en ce que ladite matrice comprend:
(a) 40 à 60 parties en poids de copolymère d'éthylène et d'acétate de vinyle,
(b) 5 à 20 parties en poids de dérivé de cellulose,
(c) 35 à 55 parties en poids d'au moins un composé choisi parmi
   - le crotamiton,
   - les 2-pyrrolidones N-substituées de formule I, 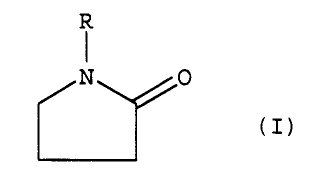 dans laquelle le groupe R représente un groupe alkyle en C₁-C₁₅, le groupe cyclohexyle ou le groupe 2-hydroxyéthyle, et
   - les alcools aliphatiques supérieurs en C₁₂-C₂₀, et
(d) 0,01 à 7 parties en poids d'une hormone choisie parmi l'ensemble constitué par les composants oestrogènes, les composants progestatifs et leurs mélanges.

Selon l'invention, on préconise également un procédé pour la préparation dudit système matriciel transdermique, ledit procédé étant caractérisé en ce qu'il comprend les étapes consistant à, soit :
- (α): mélanger l'EVA et le dérivé de cellulose sous agitation et à une température supérieure à 110°C ;
- (β): incorporer dans le mélange homogène résultant l'hormone choisie parmi l'ensemble constitué par les composants oestrogènes, les composants progestatifs et leurs mélanges, avec le crotamiton et/ou l'alcool aliphatique quand l'un au moins de ces produits intervient, sous agitation et à une température supérieure à 110°C, et homogénéiser ;
- (γ): incorporer dans le mélange homogène résultant le ou les composés 2-pyrrolidones N-substituées de formule I, s'ils interviennent, sous agitation et à une température inférieure à 110°C, et homogénéiser ;
- (δ): enduire le mélange homogène résultant de l'étape (β) ou (γ) sur un support temporaire antiadhérent, à une température comprise entre 100 et 140°C, pour obtenir un dépôt de 50 à 300 g/m² sur ledit support ; et,
- (ε): transférer la matrice ainsi obtenue sur un support final,
soit :
- (α): incorporer successivement sous agitation l'alcool aliphatique supérieur, le crotamiton et/ou la ou les 2-pyrrolidones N-substituées de formule I (si l'un ou plusieurs de ces composés sont présents dans la formulation), l'hormone choisie parmi l'ensemble constitué par les composants oestrogènes, les composants progestatifs et leur mélanges, l'EVA, le dérivé de cellulose et le solvant retenu ;
- (β): agiter le mélange ainsi obtenu, à une température inférieure à la température d'ébullition du solvant, jusqu'à homogénéisation complète dudit mélange ;
- (γ): enduire le mélange homogène résultant de l'étape (β) sur un support temporaire antiadhérent, à une température comprise entre 50 et 70°C, pour obtenir un dépôt de 50 à 300 g/m² sur ledit support ;
- (δ): chauffer l'enduction obtenue pour évaporer le solvant à une température comprise entre 40 et 80°C, en fonction du point d'ébullition de ce dernier ; et,
- (ε): transférer la matrice sèche ainsi obtenue sur un support final,

### DESSINS

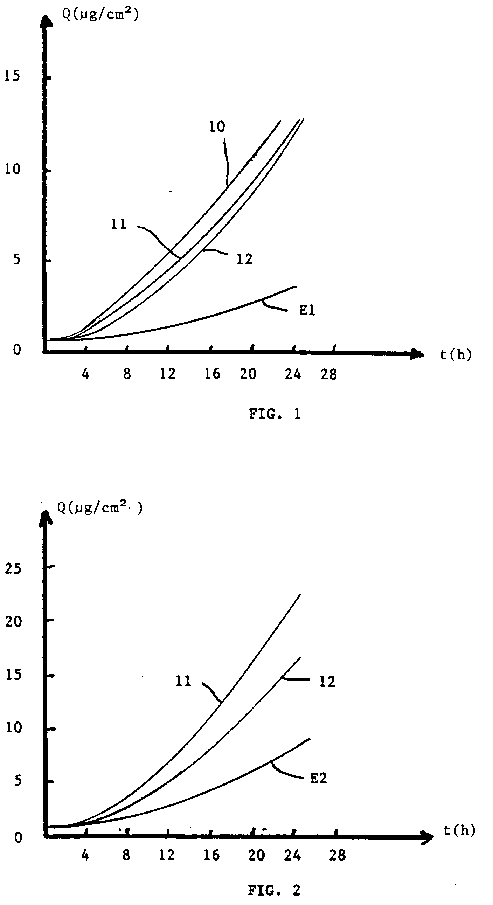

Les figures 1-4 annexées illustrent la quantité d'hormone libérée en fonction du temps pour les produits selon l'invention par rapport à un système transdermique antérieurement connu et commercialisé sous la marque ESTRAGEST® par la société CIBA-GEIGY (référencé ici E), où :
- **la figure 1** : représente la quantité libérée (Q) de 17-β-oestradiol en µg/cm² en fonction du temps (t) en heures dans le cas de systèmes combinés du type oestrogène-progestatif, les courbes 10, 11, 12 et E1 représentant respectivement les produits des exemples 10, 11, 12 selon l'invention et le dispositif ESTRAGEST® ;
- **la figure 2** : représente la quantité libérée (Q) de NETA (acétate de noréthistérone) en µg/cm² en fonction du temps (t) en heures dans le cas de systèmes combinés du type oestrogène-progestatifs, les courbes 11, 12 et E2 représentant respectivement les produits des exemples 11 et 12 selon l'invention et le dispositif ESTRAGEST® ;

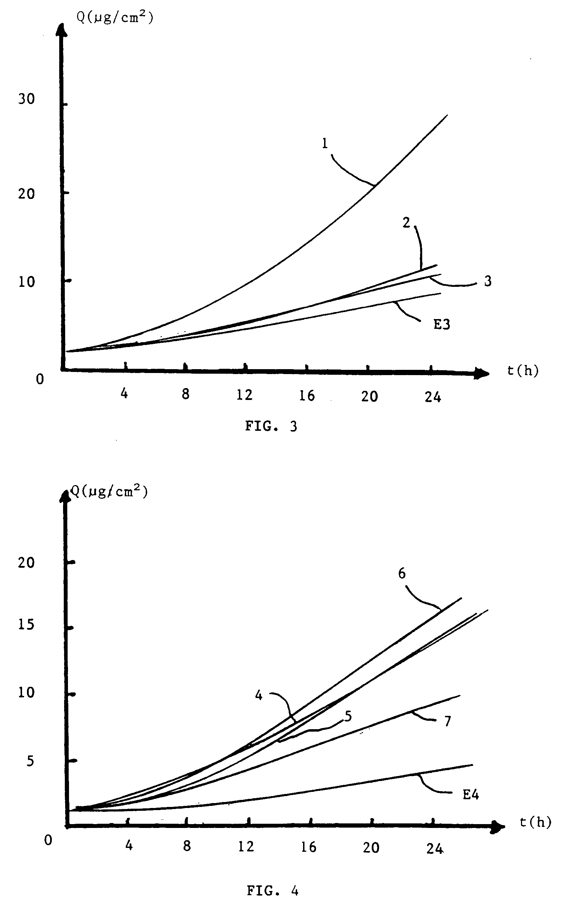

- **la figure 3** : représente la quantité libérée (Q) de NETA en µg/cm² en fonction du temps (t) en heures dans le cas de dispositifs selon l'invention contenant comme seule hormone le NETA, les courbes 1, 2, 3 et E3 représentant respectivement les produits des exemples 1, 2 et 3 selon l'invention et le dispositif ESTRAGEST® ; et,
- **la Figure 4** : représente la quantité libérée (Q) de 17-β-oestradiol en µg/cm² en fonction du temps (t) en heure dans le cas de dispositifs selon l'invention contenant comme seule hormone le 17-β-oestradiol, les courbes 4, 5, 6, 7 et E4 représentant respectivement les produits des exemples 4, 5, 6 et 7 selon l'invention et le dispositif ESTRAGEST®.

### DESCRIPTION DETAILLEE DE L'INVENTION

De préférence, on utilisera un copolymère d'éthylène et d'acétate de vinyle ayant une teneur en acétate de vinyle comprise entre 30 et 75 % en poids, en particulier de l'ordre de 45 à 60 % en poids, par rapport au poids du copolymère d'éthylène/acétate de vinyle. On pourra utiliser le cas échéant un mélange de tels EVAs présentant des poids moléculaires différents.

Parmi les alcools aliphatiques supérieurs, qui conviennent selon l'invention, on peut mentionner les monoalcools saturés ou insaturés ayant de 12 à 20 atomes de carbone, en particulier les 2-octyl-1-dodécanol, 1-hexadécanol, 1-octadécanol et 1-tétradécanol.

Par dérivé de cellulose, on entend ici les alkylcelluloses, comme par exemple la méthylcellulose, l'éthylcellulose, la propylcellulose ou la méthylpropylcellulose et les hydroxyalkylcelluloses comme par exemple l'hydroxyméthylcellulose, l'hydroxyéthylcellulose ou l'hydroxypropylcellulose.

Les 2-pyrrolidones N-substituées de formule I comprennent ici les substances dans lesquelles le groupe R représente le groupe cyclohexyle, le groupe 2-hydroxyéthyle ou un groupe alkyle de 1 à 15 atomes de carbone, comme par exemple la N-dodécyl-2-pyrrolidone, la N-octyl-2-pyrrolidone, la N-méthyl-2-pyrrolidone ou la N-éthyl-2-pyrrolidone.

Parmi les composants oestrogènes qui conviennent selon l'invention on peut citer en particulier le 17-β-oestradiol, et les dérivés de l'oestradiol, notamment les mono- et di-esters de l'oestradiol, comme par exemple le 17-acétate oestradiol, le 3,17-diacétate oestradiol, le 3-benzoate oestradiol, le 17-undécanoate oestradiol, les dérivés alkylés en position 17 de l'oestradiol tels que l'éthinyloestradiol, le 3-isopropylsulfonate éthinyloestradiol, le méthyloestradiol, le quinestrol, le mestranol et, le cas échéant, leurs mélanges.

Parmi les composants progestatifs qui conviennent selon l'invention on peut citer en particulier la progestérone, la médrogestérone et leurs dérivés (notamment l'acétate de 17-hydroxyprogestérone, l'acétate de médroxyprogestérone), la noréthistérone et ses dérivés (notamment l'acétate de 17-noréthistérone) et le norprégnane.

Selon l'invention, on utilisera de préférence comme composant oestrogène le 17-β-oestradiol et comme composant progestatif l'acétate de 17-noréthistérone (NETA).

Le support recevant la matrice pourra être tout support généralement utilisé dans les systèmes transdermiques occlusifs ou non, et imperméable aux constituants de la matrice. On préférera par exemple un support sous forme de film en polyéthylène, polypropylène, polyester, un complexe ou composite constitué de polyéthylène et d'un copolymère d'acétate de vinyle et d'éthylène ou des mousses.

De façon pratique, la surface de la matrice, qui n'est pas liée au support, pourra être recouverte d'une couche ou pellicule de protection pelable avant utilisation du dispositif ; ledit dispositif pourra être lui-même emballé dans une protection étanche comme par exemple les complexes polyéthylène-aluminium.

Grâce aux excellents rendements de libération hormonale qu'il procure, le système matriciel selon l'invention présente de nombreux avantages que l'on va maintenant exposer.

Un avantage est celui du prix de revient, très nettement abaissé par rapport aux dispositifs connus de l'art antérieur, grâce à l'utilisation d'une plus faible quantité d'hormone(s) dont le prix est élevé.

De plus, la possibilité d'utiliser moins d'oestrogènes et/ou de progestatifs tout en obtenant des quantités libérées plus importantes simplifie la mise au point et la réalisation des formulations formant la matrice des dispositifs.

On diminue aussi les risques de pollution de l'environnement par ces hormones lorsque le produit est jeté après la période de traitement.

On minimise ou supprime ainsi les problèmes de solubilité des hormones dans les EVAs ainsi que les risques d'incompatibilité chimique ou physique avec les autres constituants de la matrice. Il en est de même des problèmes de cristallisation des hormones et de l'instabilité des dispositifs au cours du temps, ces derniers phénomènes étant inacceptables pour la validation et la commercialisation de produits à visée thérapeutique tels des systèmes transdermiques.

Enfin un aspect important de l'invention est que ces résultats sont obtenus sans altérer les propriétés adhésives et cohésives de la matrice malgré l'utilisation en quantité importante de produits tels que les 2-pyrrolidones N-substituées de formule I et les alcools aliphatiques supérieurs. Ceci est possible grâce à la présence du dérivé de cellulose qui joue le rôle d'agent de cohésion et compense les effets négatifs desdits crotaminton, 2-pyrrolidones N-substituées de formule I et alcools aliphatiques supérieures. La cohésion ainsi que l'adhésion, si nécessaire, peuvent aussi être optimisés par l'utilisation de mélange d'EVAs de poids moléculaires différents.

Les systèmes transdermiques selon l'invention sont réalisés suivant les techniques généralement employées par l'homme de l'art : l'enduction en phase solvant ou selon la technique dite "hot-melt", c'est-à-dire en l'absence de solvant.

Dans les deux cas, dans le cadre d'une production industrielle, on enduit de grandes surfaces qui sont ensuite découpées pour donner des dispositifs aux dimensions adaptées, à la dose de principe actif à administrer pendant un temps déterminé.

Dans le cadre de la technique dite "hot-melt", on préconise un procédé qui comprend les étapes suivantes :
**(1)** dans un malaxeur, on incorpore l'EVA sous agitation à une température supérieure à 110°C, de préférence à la température de 130°C, puis on ajoute le dérivé de cellulose ;
**(2)** on incorpore ensuite progressivement et toujours sous agitation le principe actif puis l'alcool aliphatique supérieur et/ou le crotamiton (si l'un ou les deux sont présents dans la formulation) et on agite jusqu'à homogénéisation totale du mélange ;
**(3)** la ou les 2-pyrrolidones N-substituées de formule I qui sont plus sensibles à la chaleur sont ajoutées à une température généralement inférieure à la température du stade (1), ladite température étant déterminée selon la stabilité thermique de ces produits, soit en général aux environ de 100-110°C, on maintient l'agitation jusqu'à obtention d'un mélange parfaitement homogène ;
**(4)** on enduit le mélange homogène ainsi obtenu, à une température comprise entre 100 et 140°C, sur un support intermédiaire provisoire antiadhérent, notamment une pellicule de polyester siliconé, à raison de 50 à 300 g/m² ; et,
**(5)** on transfère ensuite la matrice obtenue à l'étape (4) sur le support final choisi.

Dans le cadre de la technique dite "en phase solvant" on préconise un procédé pour la préparation d'un système matriciel auto-adhésif selon l'invention qui comprend les étapes suivantes :
**(1)** dans un malaxeur on incorpore sous agitation successivement l'alcool aliphatique supérieur, le crotamiton et/ou la ou les 2-pyrrolidones N-substituées de formule I (si l'un ou plusieurs de ces composés sont présents dans la formulation), le ou les principes actifs, l'EVA, le dérivé de cellulose et le solvant comme par exemple l'acétate d'éthyle ;
**(2)** on agite le mélange obtenu à une température comprise entre 50 et 70°C (inférieure à la température d'ébullition du solvant choisi) jusqu'à homogénéisation totale du mélange ;
**(3)** on enduit le mélange homogène ainsi obtenu, à une température comprise entre 50 et 70°C, sur un support intermédiaire provisoire antiadhérent, notamment une pellicule de polyester siliconée à raison de 50 à 300 g/m² ;
**(4)** on évapore le solvant en chauffant à une température comprise entre 40 et 110°C, de préférence 60 à 80°C, en fonction du point d'ébullition de ce dernier ; et,
**(5)** on transfère la matrice sèche obtenue à l'étape (4) sur le support final choisi.

Le nouveau dispositif matriciel auto-adhésif selon l'invention est en particulier utile pour le traitement de l'ostéoporose, des symptômes de la ménopause et des risques cardio-vasculaires qui en découlent, dans le cadre d'une thérapie dite "de remplacement des hormones déficitaires" ainsi que pour tout traitement reposant sur l'administration d'oestrogènes et/ou de progestatifs par voie transdermique.

### MEILLEUR MODE

Le meilleur mode de mise en oeuvre de l'invention consiste à faire appel à un système matriciel transdermique dont la matrice contient pour un total de 100 parties en poids :
**(a)** 46 parties en poids d'EVA,
**(b)** 10 parties en poids d'éthylcellulose,
**(c1)** 31,5 parties en poids de 2-octyl-1-dodécanol,
**(c2)** 10,5 parties en poids de N-dodécyl-2-pyrrolidone,
   et
**(d)** 2 parties en poids de 17-β-oestradiol,
d'une part, ou
**(a)** 45 parties en poids d'EVA,
**(b)** 10 parties en poids d'éthylcellulose,
**(c1)** 30,75 parties en poids de 2-octyl-1-dodécanol,
**(c2)** 10,25 parties en poids de N-dodécyl-2-pyrrolidone, et
**(d)** 4 parties en poids d'acétate de noréthistérone,
d'autre part, ou
**(a)** 42 parties en poids d'EVA,
**(b)** 15 parties en poids d'éthylcellulose,
**(c1)** 28 parties en poids de 2-octyl-1-dodécanol,
**(c2)** 10 parties en poids de N-dodécyl-2-pyrrolidone,
**(d1)** 2 parties en poids de 17-β-oestradiol, et,
**(d2)** 3 parties en poids d'acétate de noréthistérone

Dans ces formulations, l'EVA utilisé présente avantageusement une teneur en acétate de vinyle de 35 à 60 % en poids par rapport au poids dudit EVA.

D'autres avantages et caractéristiques de l'invention seront mieux compris à la lecture de la description qui va suivre d'exemples de réalisation et d'essais comparatifs.

Bien entendu l'ensemble de ces éléments n'est nullement limitatif mais est donné à titre d'illustration.

Par commodité, dans ce qui suit les abréviations suivantes ont été utilisées :
- **EVA :**: copolymère d'éthylène et d'acétate de vinyle
- **Es :**: 17-β-oestradiol
- **NETA :**: acétate de noréthistérone

### Exemple 1

Dans un bécher de 250 ml contenant 40 g d'acétate d'éthyle on introduit par fractions, sous agitation et en chauffant à 60°C, 3 g d'ETHOCEL® (éthylcellulose commercialisée par la société DOW CHEMICAL), puis 6,75 g d'ELVAX® 46L et 6,75 g d'ELVAX® 46 (EVAs commercialisés par la société DU PONT). On agite ainsi pendant au moins 30 minutes. On introduit alors toujours à 60°C 12,3 g de SURFADONE® LP 300 (N-dodécyl-2-pyrrolidone commercialisée par la société GAF CORPORATION) et 1,2 g de NETA préalablement dissous dans 5 g de tétrahydrofuranne. On agite environ 30 minutes à 60°C puis on laisse dégazer le mélange obtenu. On enduit ce dernier à 60°C de façon à former un dépôt de (100 ± 10) g/m² sur un support temporaire en polyester siliconé. On laisse alors le produit enduit, ainsi obtenu, à température ambiante (15-20°C) pendant au minimum 15 heures pour que le solvant s'évapore. On transfère alors la matrice ainsi obtenue sur un support final en polyéthylène. On découpe à la taille désirée et conditionne les produits ainsi découpés dans des sachets thermosoudables.

On obtient un système dans lequel la quantité initiale en NETA est de 400,3 µg/cm².

### Exemple 2

On procède de façon identique à l'exemple 1, mais en utilisant ici 40 g d'acétate d'éthyle, 3 g d'ETHOCEL®, 6,75 g d'ELVAX® 46L, 6,75 g d'ELVAX® L, 10,8 g d'EUTANOL® G (2-octyl-dodécanol commercialisé par la société HENKEL), 1,5 g de SURFADONE® LP 300 et 1,2 g de NETA dissous dans 5 g de tétrahydrofuranne. On enduit de façon à obtenir un dépôt matriciel de (100 ± 10) g/m².

On obtient un système dans lequel la quantité initiale en NETA est de 400,3 µg/cm².

### Exemple 3

On procède de façon identique à l'exemple 1, mais en utilisant ici 40 g d'acétate d'éthyle, 2,96 g d'ETHOCEL® 6,75 g d'ELVAX® 46L, 6,75 g d'ELVAX® 46, 6,17 g d'EUTANOL® G, 6,17 g de crotamiton [(N-éthyl-N(2-méthylphényl)-2-buténamide, produit commercialisé par la société BOEHRINGER INGELHEIM)] et 1,2 g de NETA dissous dans 5 g de tétrahydrofuranne.On enduit de façon à obtenir un dépôt matriciel de 100 ± 10) g/m².

On obtient un système dans lequel la quantité initiale en NETA est de 408,8 µg/cm².

### Exemple 4

On procède de façon analogue à l'exemple 1, mais en utilisant ici 3 g d'ETHOCEL® 6,87 g d'ELVAX® 46L, 6,87 g d'ELVAX® 46, 12,66 g d'EUTANOL® G, 40 g d'acétate d'éthyle et 0,6 g de 17-β-oestradiol dissous dans 5 g de tétrahydrofuranne. On enduit de façon à obtenir un dépôt matriciel de (100 ± 10) g/m².

On obtient un système dans lequel la quantité initiale en 17-β-oestradiol est de 200 µg/cm².

### Exemple 5

On procède de façon analogue à l'exemple 4, mais on remplace ici l'EUTANOL® G par la même quantité de SURFADONE® LP 300. On enduit de façon à obtenir un dépôt matriciel de (100 ± 10) g/m².

On obtient un système dans lequel la quantité initiale en 17-β-oestradiol est de 200 µg/cm².

### Exemple 6

On procède de façon analogue à l'exemple 1, mais en utilisant 3 g d'ETHOCEL®, 6,87 g d'ELVAX® 46L, 6,87 g d'ELVAX® 46, 40 g d'acétate d'éthyle, 11,16 g d'EUTANOL® G, 1,5 g de SURFADONE® LP 300 et 0,6 g de 17-β-oestradiol dissous dans 5 g de tétrahydrofuranne. On enduit de façon à obtenir un dépôt matriciel de (100 ± 10) g/m².

On obtient un système dans lequel la quantité initiale en 17-β-oestradiol est de 205,4 µg/cm².

### Exemple 7

On procède de façon analogue à l'exemple 1, mais en utilisant 3 g d'ETHOCEL® 6,87 g d'ELVAX® 46L, 6,87 g d'ELVAX® 46, 40 g d'acétate d'éthyle, 6,33 g d'EUTANOL® G, 6,33 g de crotamiton et 0,6 g de 17-β-oestradiol dissous dans 5 g de tétrahydrofuranne. On enduit de façon à obtenir un dépôt matriciel de (100 ± 10) g/m².

On obtient un système dans lequel la quantité initiale en 17-β-oestradiol est de 200,2 µg/cm².

### Exemple 8

On procède de façon analogue à l'exemple 7, mais ici on remplace le crotamiton par une même quantité de SURFADONE® LP 300. On enduit de façon à obtenir un dépôt matriciel de (100 ± 10) g/m².

On obtient un système dans lequel la quantité initiale en 17-β-oestradiol est de 200 µg/cm²

### Exemple 9

On procède de façon analogue à l'exemple 1, mais en utilisant 2,91 g d'ETHOCEL®, 6,60 g d'ELVAX® 46L, 6,60 g d'ELVAX® 46, 12,09 g d'EUTANOL® G, 40 g d'acétate d'éthyle, 1,2 g de NETA et 0,6 g de 17-β-oestradiol dissous dans 5 g de tétrahydrofuranne. On enduit de façon à obtenir un dépôt matriciel de (100 ± 10) g/m².

On obtient un système dans lequel la quantité initiale en NETA est de 400 µg/cm² et celle de 17-β-oestradiol de 200 µg/cm².

### Exemple 10

On procède de façon identique à l'exemple 1, mais en utilisant 2,9 g d'ETHOCEL®, 6,60 g d'ELVAX® 46L, 6,60 g d'ELVAX® 46, 9,68 g d'EUTANOL® G, 40 g d'acétate d'éthyle, 2,42 g de SURFADONE® LP 300, et une solution de 1,2 g de NETA plus 0,6 g de 17-β-oestradiol dissous dans 5 g de tétrahydrofuranne. On enduit de façon à obtenir un dépôt matriciel de (100 ± 10) g/m².

On obtient un système dans lequel la quantité initiale en NETA est de 399,6 µg/cm² et celle de 17-β-oestradiol de 200 µg/cm².

### Exemple 11

On procède de façon analogue à l'exemple 10, mais ici on utilise respectivement 2,42 g d'EUTANOL® G, et 9,68 g de SURFADONE® LP 300. On enduit de façon à obtenir un dépôt matriciel de (100 ± 10) g/m².

On obtient un système dans lequel la quantité initiale en NETA est de 400 µg/cm² et celle de 17-β-oestradiol de 200 µg/cm².

### Exemple 12

On procède de façon analogue à l'exemple 10, mais ici on utilise respectivement 6,05 g d'EUTANOL® G, et 6,05 g de SURFADONE® LP 300. On enduit de façon à obtenir un dépôt matriciel de (100 ± 10) g/m².

On obtient un système dans lequel la quantité initiale en NETA est de 400 µg/cm² et celle de 17-β-oestradiol de 200 µg/cm².

### Exemple 13

On procède de façon analogue à l'exemple 6, mais en utilisant 10,35 g d'ELVAX® 46L, 3,45 g d'ELVAX® 46, 3 g d'ETHOCEL®, 40 g d'acétate d'éthyle, 9,45 g d'EUTANOL® G, 3,15 g de SURFADONE® LP 300, et 0,6 g de 17-β-oestradiol dissous dans 5 g de tétrahydrofuranne. On enduit de façon à obtenir un dépôt matriciel de (100 ± 10) g/m².

On obtient un système dans lequel la quantité initiale en 17-β-oestradiol est de 190,3 µg/cm².

### Exemple 14

On procède de façon analogue à l'exemple 2, mais en utilisant 10,13 g d'ELVAX® 46L, 3,38 g d'ELVAX® 46, 3 g d'ETHOCEL®, 40 g d'acétate d'éthyle, 9,23 g d'EUTANOL® G, 3,07 g de SURFADONE® LP 300, et 1,2 g de NETA dissous dans 5 g de tétrahydrofuranne. On enduit de façon à obtenir un dépôt matriciel de (110 ± 10) g/m².

On obtient un système dans lequel la quantité initiale en NETA est de 466,7 µg/cm².

### Exemple 15

Dans un mélangeur, on incorpore successivement 130 g de SURFADONE® LP 300, 364 g d'EUTANOL® G, 26 g de 17-β-oestradiol, 39 g de NETA, 546 g de LEVAPREN® 450 P (copolymère EVA contenant 45 % en poids d'acétate de vinyle commercialisé par la société BAYER) et 195 g d'ETHOCEL®. On agite le mélange progressivement et on incorpore 1300 g d'acétate d'éthyle. On chauffe ce mélange à 65°C en agitant jusqu'à dissolution complète des constituants puis on laisse dégazer le mélange résultant. On enduit le mélange ainsi obtenu, entre 50 et 60°C, sur un support temporaire en polyester siliconé de façon à former un dépôt de (100 ± 10) g/m². On sèche le revêtement ainsi obtenu à une température comprise entre 60 et 80°C pour évaporer le solvant. On transfère la matrice ainsi obtenue sur un support final en polyéthylène.

On obtient un système dans lequel la quantité initiale en 17-β-oestradiol est de 192 µg/cm². la quantité initiale en NETA est de 291 µg/cm².

### Exemple comparatif CP1

On prépare un dispositif du type de ceux décrits dans la demande de brevet publiée FR-A-2 612 785.

On introduit dans un malaxeur de 5 litres 1610 g de LEVAPREN® 450 (EVA ayant une teneur en motifs acétate de vinyle de 45 % commercialisé par la société BAYER) et 1070 g d'EUTANOL® G (2-octyldodécanol commercialisé par la société HENKEL). On chauffe à 140°C et on additionne au mélange, par petites fractions, 262,5 g d'ETHOCEL® 20 (éthylcellulose de viscosité égale à 2x10⁻² Pa.s). Après homogénéisation du milieu, on ajoute 540 g d'EUTANOL® G. On homogénéise l'ensemble pendant 0,5 heure et on laisse reposer pendant 24 heures. On porte ensuite la masse à 60°C pendant 0,5 heure et on additionne une solution de 17,5 g de dipropylèneglycol (DPG) et 175 g de 17-β-oestradiol dans 875 g d'éthanol anhydre. On homogénéise l'ensemble pendant 1 heure sans chauffage. On enduit cette masse sur papier siliconé de 105 mm de large à une température de 60°C à raison de (120 ± 10) g/m². Après passage du papier siliconé enduit à 80°C pour évaporer l'éthanol, on transfère la matrice sur un support final en polyéthylène. On découpe des formes aux dimensions désirées et conditionne en sachets aluminium/polyéthylène thermosoudés.

On obtient un système dans lequel la quantité initiale en 17-β-oestradiol est de 565 µg/cm².

Les rendements des dispositifs selon l'invention sont déterminés à partir des mesures de quantités d'hormone(s) libérées en 24 heures sur un modèle de peau ex-vivo.

Pour cela on a réalisé des tests de perméation ex-vivo sur peau abdominale de souris "nude" mâle selon le protocole suivant.

La mesure des quantités d'hormone(s) libérées par un dispositif transdermique d'une surface de 2,54 cm², préalablement découpé à l'emporte pièce et déposé sur un disque de 3,14 cm² de peau abdominale de souris "nude" mâle, est effectuée dans une cellule statique en verre thermostatée à 37°C et ayant un compartiment récepteur d'un volume de 11,5 ml, ce compartiment récepteur contenant comme phase réceptrice un mélange sérum physiologique/PEG₄₀₀ (75/25) (V/V).

On effectue des prélèvements dans les solutions réceptrices à 2, 4, 6, 8, 12, 16, 20 et 24 heures, et on les dose par chromatographie liquide.

Compte tenu de la variabilité des résultats liés à la perméabilité intrinsèque des échantillons de peau, chaque essai de perméation pour un échantillon de dispositif transdermique est réalisé sur un nombre minimum de 3 à 5 échantillons de peau.

Le résultat, qui est donné, est la moyenne obtenue pour chaque dispositif à partir de ces essais. Le rapport entre cette valeur moyenne des quantités d'hormone(s) libérées en fin de cinétique à 24 heures et la quantité initiale d'hormone(s) contenue dans le dispositif permet d'évaluer le rendement à 24 heures des systèmes transdermiques selon l'invention.

Dans un but comparatif on a déterminé de la même façon les quantités d'hormones libérées à 24 heures du seul produit aujourd'hui disponible comprenant à la fois un oestrogène et un progestatif, à savoir le dispositif commercialisé sous la marque ESTRAGEST® par la société CIBA-GEIGY. C'est d'ailleurs le seul système transdermique commercial qui contienne un composant progestatif.

Le dispositif ESTRAGEST® est formé de deux réservoirs accolés contenant au total 10 mg de 17-β-oestradiol et 30 mg de NETA, chaque réservoir contenant un mélange de 5 mg de 17-β-oestradiol et 15 mg de NETA.

Les mesures de perméation cutanée sont effectuées suivant le même protocole sur un seul des deux réservoirs placé sur un échantillon de peau de 3,14 cm². Les quantités initiales d'hormone(s) contenues dans ce réservoir sont ramenées à la quantité initiale d'hormone(s) par unité de surface exprimée en µg/cm².

Le rapport de la valeur moyenne des quantités de 17-β-oestradiol ou de NETA libérées en 24 heures à la quantité initiale contenue dans le réservoir permet d'obtenir les rendements à 24 heures en 17-β-oestradiol ou de NETA.

Les résultats obtenus ont été consignés dans les tableaux I et II ci-après.

On a aussi réalisé des tests comparatifs de perméation ex-vivo sur peau abdominale de souris "nude" mâle vis à vis des systèmes matriciels antérieurement connus, en particulier ceux décrits dans la demande de brevet FR-A-2 612 785 précitée.

Dans ce cas les mesures sont effectuées d'après le protocole décrit ci-dessus mais ici pour se conformer aux conditions opératoires décrites dans ladite demande de brevet, on applique un dispositif transdermique selon l'invention d'une surface de 2 cm² et on utilise comme phase réceptrice un mélange sérum physiologique/ éthanol/PEG₄₀₀ (64/20/16) (V/V).

De plus dans ce cas, on a effectué des prélèvements réguliers jusqu'à 48 heures. On a ainsi toujours calculé les rendements à 24 et 48 heures comme étant le rapport entre la valeur moyenne de la quantité de 17-β-oestradiol libéré et la quantité initiale 17-β-oestradiol contenue dans le dispositif de cette demande de brevet. C'est l'exemple comparatif 1 (CP1).

L'ensemble des résultats, qui ont été obtenus ont été consignés dans le tableau III ci-après.

Dans le cas d'une matrice contenant à la fois du 17-β-oestradiol et du NETA, le tableau I illustre les avantages des systèmes selon l'invention vis à vis du produit ESTRAGEST® précité.

On constate dans ce cas que, comme le montrent les courbes (10-11), (11-12) et (1-2) des figures 1 à 3, on a toujours des quantités libérées d'hormones par les dispositifs selon l'invention pour le 17-β-oestradiol comme pour le NETA qui sont significativement supérieures à celles du système ESTRAGEST®, ceci avec des quantités initiales moindres, respectivement 8 et 12 fois inférieures.

De plus comme le montre le tableau I, on a dans le cas du 17-β -oestradiol des rendements en moyenne 30 fois supérieurs à celui d'ESTRAGEST®, et dans le cas du NETA des rendements 14 à 33 fois supérieurs à ceux d'ESTRAGEST®.

Ces différences importantes démontrent à nouveau les avantages de l'invention qui permet de réaliser des gains de coûts importants en utilisant moins de produits dans un but thérapeutique recherché, d'éviter les problèmes éventuels de cristallisation dans la matrice, de simplifier la mise au point et de fabriquer des systèmes de tailles plus réduites.

De même on a des résultats analogues dans le cas de systèmes contenant le 17-β-oestradiol seul ou le NETA seul comme cela est illustré par les figures 3 et 4, d'une part, et le tableau II, d'autre part.

Ainsi la figure 4 montre que pour les produits des exemples 4, 5, 6 et 7 selon l'invention les quantités de 17-β-oestradiol libérées lors de tests de perméation cutanée ex-vivo sur peau abdominale de souris "nude" sont toujours supérieures à celles libérées par ESTRAGEST®.

De même la figure 3 montre que, dans le cas du NETA utilisé seul dans la matrice (exemples 1, 2 et 3), par comparaison avec la libération de NETA dans ESTRAGEST®, les dispositifs selon l'invention ont toujours des quantités libérées supérieures, en particulier dans le cas du produit de l'exemple 1, et ceci malgré des concentrations 12 fois inférieures.

Les résultats du tableau II montrent que l'on a toujours des quantités libérées de 17-β-oestradiol et de NETA supérieures à celle du dispositif ESTRAGEST®.

L'analyse des rendements du tableau II montre que par rapport au dispositif ESTRAGEST® on a respectivement des rendements pour le 17-β-oestradiol 33 fois supérieurs pour les produits des exemples 4 et 5, 35 fois supérieurs pour le produit de l'exemple 6 et 21 fois supérieurs pour le produit de l'exemple 7.

De même pour le NETA on a respectivement des rendements 17 fois, 16 fois et même 42 fois supérieurs pour les produits des exemples 2, 3 et 1.

On retrouve, en comparant le produit ESTRAGEST® avec les produits des autres exemples selon l'invention, des résultats identiques à ceux du tableau I.

Les résultats du tableau III mettent également en évidence les avantages des dispositifs selon l'invention par rapport aux systèmes décrits dans la demande de brevet FR-A-2 612 785 illustrés ici par l'exemple comparatif CP1.

On constate à nouveau que les rendements obtenus avec les produits selon l'invention sont toujours supérieurs. Ils sont environ 2 fois supérieurs à 24 heures et 1,7 fois supérieurs à 48 heures. Compte tenu de la variabilité des résultats obtenus avec le modèle cutanée choisie. on a donc des résultats comparables.

On a enfin réalisé toujours sur peau abdominale de souris "nude" en utilisant le même protocole avec comme phase réceptrice le mélange sérum physiologique/PEG₄₀₀ (75/25) (V/V) des mesures de quantités libérées et de rendements à 24 heures avec des dispositifs suivant l'invention, où le rapport pondéral entre les deux copolymères d'EVAs de la matrice est de 3 (produits des exemples 13 et 14) et diffère du rapport des exemples précédents qui était toujours de 1. On a aussi utilisé un dispositif suivant l'invention qui ne contient qu'un seul copolymère EVA (exemple 15). Les résultats obtenus avec les produits de ces 3 exemples, comparés au produit ESTRAGEST® sont rassemblés dans le tableau IV.

On constate à nouveau que, aussi bien avec le 17-β-oestradiol seul (exemple 13), qu'avec le NETA seul (exemple 14), ou avec un mélange de ces 2 hormones (exemples 15), on trouve toujours des quantités libérées en 24 heures et des rendements à 24 heures du même ordre que précédemment et toujours très supérieurs à ceux d'ESTRAGEST®.
- On a ainsi :: pour l'exemple 13 un rendement 27 fois supérieur pour le 17-β-oestradiol,
pour l'exemple 14 un rendement 22 fois supérieur pour le NETA,
pour l'exemple 15 un rendement 20 supérieur pour le NETA et 32 fois pour le 17-β-oestradiol.

**TABLEAU I**

| Comparaison sur peau abdominale de souris "nude" mâle. | | | | | | | | | | |
|---|---|---|---|---|---|---|---|---|---|---|
| | **Ex 9** | | **Ex 10** | | **Ex 11** | | **Ex 12** | | **ESTRAGEST®** | |
| | **Es** | **NETA** | **Es** | **NETA** | **Es** | **NETA** | **Es** | **NETA** | **Es** | **NETA** |
| **Q**_{**0**} | 200 | 399,6 | 200 | 400,6 | 200 | 400 | 200,2 | 400 | 1604 | 4740 |
| **Q**_{**24**} | 13,24± 2,2 | 8,99±1 ,2 | 13,48± 1,5 | 11,86± 1,6 | 12,73± 2,6 | 21,58± 4,7 | 12,41± 0,6 | 15,29± 2,6 | 3,37±0 ,97 | 7,58±1 ,5 |
| **R** | 6,62 | 2,25 | 6,74 | 2,96 | 6,36 | 5,4 | 6,2 | 3,82 | 0,21 | 0,16 |
| Q₂₄ : Quantité de 17-β-oestradiol ou de NETA libérée en 24 heures exprimée en µg/cm² Q₀ : Quantité initiale de 17-β-oestradiol ou de NETA exprimée en µg/cm² R : Rendement exprimé en pourcentage (R = 100.Q₂₄/Q₀). | | | | | | | | | | |

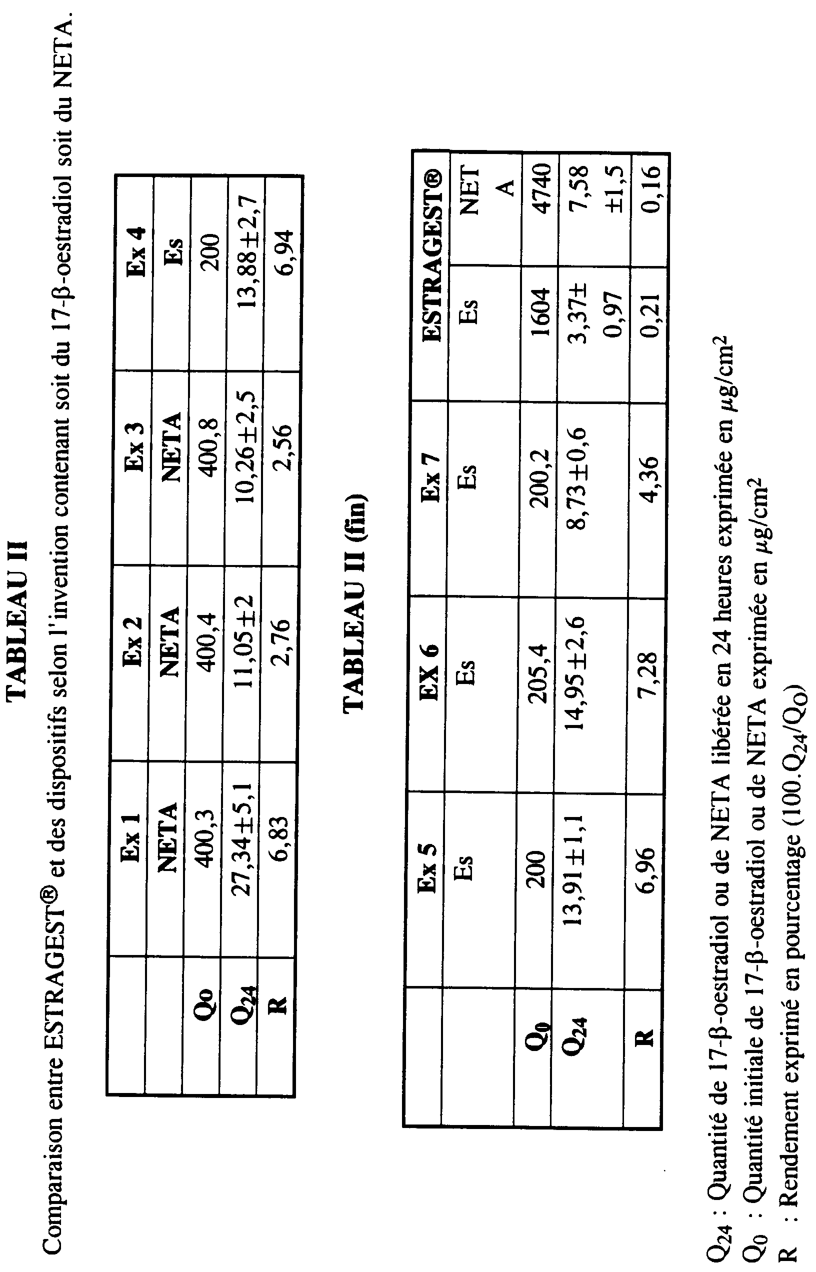

**TABLEAU III**

| Comparaison entre les dispositifs selon l'invention et le dispositif décrit dans la demande de brevet FR-A-2-612 785. | | | | |
|---|---|---|---|---|
| | **CP1** | **Ex 5** | **Ex 6** | **Ex 8** |
| **Qo** | 565 | 200 | 205,4 | 200 |
| **Q**_{**24**} | 64,34±20,8 | 43,65±4,7 | 48,15±2,6 | 41,8±5,5 |
| **R** _{**24**} | 11,4 | 21,8 | 24,1 | 20,9 |
| **Q**_{**48**} | 137,4±31,5 | 76,38±7,5 | 89,52±5,2 | 81,15±9,8 |
| **R**_{**48**} | 24,2 | 38,2 | 44,8 | 40,6 |
| Qo = quantité initiale de 17-β-oestradiol exprimée en µg/cm² Q₂₄ = quantité de 17-β-oestradiol libérée en 24 heures, exprimée en µg/cm² Q₄₈ = quantité de 17-β-oestradiol libérée en 48 heures, exprimée en µg/cm² R₂₄ = rendement en pourcentage à 24 heures R₄₈ = rendement en pourcentage à 48 heures | | | | |

**TABLEAU IV**

| Comparaison entre ESTRAGEST® et les dispositifs selon l'invention contenant 17-β-oestradiol et/ou du NETA à base de copolymères EVA différents. | | | | | | |
|---|---|---|---|---|---|---|
| | **Ex 13** | **Ex 14** | **Ex 15** | | **ESTRAGEST®** | |
| | **Es** | **NETA** | **Es** | **NETA** | **Es** | **NETA** |
| **Q**_{**0**} | 190,3 | 466,7 | 192 | 291 | 1604 | 4740 |
| **Q**_{**24**} | 11,04±1,7 | 16,8±2,6 | 12,9±2,6 | 9,3±2,6 | 3,37±0,97 | 7,58±1,5 |
| **R** | 5,8 | 3,6 | 6,72 | 3,2 | 0,21 | 0,16 |
| Qₒ = quantité de 17-β-oestradiol et/ou de NETA exprimée en µg/cm² Q₂₄ = quantité initiale de 17-β-oestradiol et/ou de NETA libérée en 24 heures exprimée en µg/cm² R = rendement exprimé en pourcentage (100.Q₂₄/Q₀). | | | | | | |

## Revendications

1. Système matriciel transdermique auto-adhésif pour l'administration par voie percutanée d'une hormone, ledit système, qui comprend un support et une matrice auto-adhésive, étant caractérisé en ce que ladite matrice comprend :
**(a)** 40 à 60 parties en poids de copolymère d'éthylène et d'acétate de vinyle,
**(b)** 5 à 20 parties en poids de dérivé de cellulose,
**(c)** 35 à 55 parties en poids d'au moins un composé choisi parmi
- le crotamiton,
- les 2-pyrrolidones N-substituées de formule I, 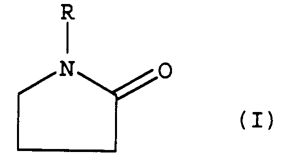 dans laquelle le groupe R représente un groupe alkyle en C₁-C₁₅, le groupe cyclohexyle ou le groupe 2-hydroxyéthyle, et
- les alcools aliphatiques supérieurs en C₁₂-C₂₀, et
**(d)** 0,01 à 7 parties en poids d'une hormone choisie parmi l'ensemble constitué par les composants oestrogènes, les composants progestatifs et leurs mélanges.

2. Système selon la revendication 1 caractérisé en ce que l'alcool aliphatique supérieur est le 2-octyl-1-dodécanol.

3. Système selon la revendication 1 caractérisé en ce que le composé de formule I est une N-alkyl-2-pyrrolidone, où le groupe alkyle est en C₁-C₁₅, de préférence la N-dodécyl-2-pyrrolidone.

4. Système selon les revendications 1 à 3 caractérisé en ce que le copolymère d'éthylène et d'acétate de vinyle a une teneur en acétate de vinyle comprise entre 30 et 75 % en poids par rapport au poids dudit copolymère.

5. Système selon l'une quelconque des revendications 1 à 4 caractérisé en ce que l'hormone est un composant oestrogène, de préférence le 17-β-oestradiol.

6. Système selon l'une quelconque des revendications 1 à 4 caractérisé en ce que l'hormone est un composant progestatif, de préférence l'acétate de noréthistérone.

7. Système selon la revendication 1, caractérisé en ce que l'hormone est un mélange constitué d'un composant oestrogène et d'un composant progestatif.

8. Système selon l'une quelconque des revendications 1 à 7, caractérisé en ce que sa matrice comprend, pour un total de 100 parties en poids :
**(a)** 46 parties en poids d'EVA,
**(b)** 10 parties en poids d'éthylcellulose,
**(c1)** 31,5 parties en poids de 2-octyl-1-dodécanol,
**(c2)** 10,5 parties en poids de N-dodécyl-2-pyrrolidone, et
**(d)** 2 parties en poids de 17-β-oestradiol.

9. Système selon l'une quelconque des revendications 1 à 7, caractérisé en ce que sa matrice comprend, pour un total de 100 parties en poids :
**(a)** 45 parties en poids d'EVA,
**(b)** 10 parties en poids d'éthylcellulose,
**(c1)** 30,75 parties en poids de 2-octyl-1-dodécanol,
**(c2)** 10,25 parties en poids de N-dodécyl-2-pyrrolidone, et
**(d)** 4 parties en poids d'acétate de noréthistérone.

10. Système selon l'une quelconque des revendications 1 à 7, caractérisé en ce que sa matrice comprend, pour un total de 100 parties en poids :
**(a)** 42 parties en poids d'EVA,
**(b)** 15 parties en poids d'éthylcellulose,
**(c1)** 28 parties en poids de 2-octyl-1-dodécanol,
**(c2)** 10 parties en poids de N-dodécyl-2-pyrrolidone,
**(d1)** 2 parties en poids de 17-β-oestradiol, et
**(d2)** 3 parties en poids d'acétate de noréthistérone.

11. Procédé pour la préparation d'un système matriciel transdermique suivant l'une quelconque des revendications 1 à 9, ledit procédé étant caractérisé en ce qu'il comprend les étapes consistant à :
**(**α**)** mélanger l'EVA et le dérivé de cellulose sous agitation et à une température supérieure à 110°C ;
**(**β**)** incorporer dans le mélange homogène résultant l'hormone choisie parmi l'ensemble constitué par les composants oestrogènes, les composants progestatifs et leurs mélanges, avec le crotamiton et/ou l'alcool aliphatique quand l'un au moins de ces produits intervient, sous agitation et à une température supérieure à 110°C, et homogénéiser ;
**(**γ**)** incorporer dans le mélange homogène résultant le ou les composés 2-pyrrolidones N-substituées de formule I, s'ils interviennent, sous agitation et à une température inférieure à 110°C, et homogénéiser ;
**(**δ**)** enduire le mélange homogène résultant de l'étape (β) ou (γ) sur un support temporaire antiadhérent, à une température comprise entre 100 et 140°C, pour obtenir un dépôt de 50 à 300 g/m² sur ledit support ; et,
**(**ε**)** transférer la matrice ainsi obtenue sur un support final.

12. Procédé pour la préparation d'un système matriciel transdermique suivant l'une quelconque des revendications 1 à 5, ledit procédé étant caractérisé en ce qu'il comprend les étapes consistant à :
**(**α**)** incorporer successivement sous agitation l'alcool aliphatique supérieur, le crotamiton et/ou la ou les 2-pyrrolidones N-substituées de formule I (si l'un ou plusieurs de ces composés sont présents dans la formulation), l'hormone choisie parmi l'ensemble constitué par les composants oestrogènes, les composants progestatifs et leur mélanges, l'EVA, le dérivé de cellulose et le solvant retenu ;
**(**β**)** agiter le mélange ainsi obtenu, à une température inférieure à la température d'ébullition du solvant, jusqu'à homogénéisation dudit mélange ;
**(**γ**)** enduire le mélange homogène résultant de l'étape (β) sur un support temporaire antiadhérent, à une température comprise entre 50 et 70°C, pour obtenir un dépôt de 50 à 300 g/m² sur ledit support ;
**(**δ**)** chauffer l'enduction obtenue pour évaporer le solvant à une température comprise entre 40 et 80°C, en fonction du point d'ébullition de ce dernier ;
**(**ε**)** transférer la matrice sèche ainsi obtenue sur un support final.

## Claims

1. A self-adhesive transdermal matrix system for the administration by percutaneous route of a hormone, said system, which comprises a support and self-adhesive matrix, wherein said matrix comprises :
**(a)** 40 to 60 parts by weight of an ethylene/vinyl acetate copolymer,
**(b)** 5 to 20 parts by weight of a cellulosic material,
**(c)** 35 to 55 parts by weight of at least one compound selected from the group consisting of:
- crotamiton,
- N-substituted 2-pyrrolidones fo the formula I, 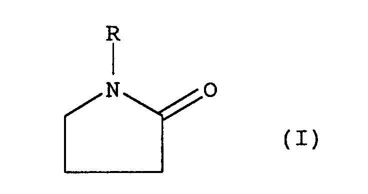 in which the R group represents a (C₁-C₁₅)-alkyl, cyclohexyl or 2-hydroxyethyl group, and
- higher C₁₂-C₂₀ aliphatic alcohols, and
(d) 0,01 to 7 parts by weight of a hormone selected from the group consisting of oestrogen components, progestogen components and mixtures thereof.

2. A system according to claim 1 wherein the higher aliphatic alcohol is 2-octyl-1-dodecanol.

3. A system according to claim 1 wherein the compound of the formula I is an N-alkyl-2-pyrrolidone, in which the alkyl group contains from 1 to 15 carbon atoms, the preferred compound being N-dodecyl-2-pyrrolidone.

4. A system according to claims 1 to 3 wherein the ethylene/vinyl acetate copolymer has a vinyl acetate content of between 30 and 75% by weight with respect to the weight of said copolymer.

5. A system according to any one of claims 1 to 4, wherein the hormone is an oestrogen component, preferably 17-β-oestradiol.

6. A system according to any one of claims 1 to 4, wherein the hormone is a progestogen component, preferably norethisterone acetate.

7. A system according to claim 1, wherein the hormone is a mixture consisting of an oestrogen component and a progestogen component.

8. A system according to any one of claims 1 to 7, wherein the matrix comprises, per total 100 parts by weight :
**(a)** 46 parts by weight of EVA,
**(b)** 10 parts by weight of ethylcellulose,
**(c1)** 31.5 parts by weight of 2-octyl-1-dodecanol,
**(c2)** 10.5 parts by weight of N-dodecyl-2-pyrrolidone, and
**(d)** 2 parts by weight of 17-β-oestradiol.

9. A system according to any one of claims 1 to 7, wherein the matrix comprises, per total 100 parts by weight :
**(a)** 45 parts by weight of EVA,
**(b)** 10 parts by weight of ethylcellulose,
**(c1)** 30.75 parts by weight of 2-octyl-1-dodecanol,
**(c2)** 10.25 parts by weight of N-dodecyl-2-pyrrolidone, and
**(d)** 4 parts by weight of norethisterone acetate.

10. A system according to any one of claims 1 to 7, wherein the matrix comprises, per total 100 parts by weight :
**(a)** 42 parts by weight of EVA,
**(b)** 15 parts by weight of ethylcellulose,
**(c1)** 28 parts by weight of 2-octyl-1-dodecanol,
**(c2)** 10 parts by weight of N-dodecyl-2-pyrrolidone,
**(d1)** 2 parts by weight of 17-β-oestradiol, and
**(d2)** 3 parts by weight of norethisterone acetate.

11. A method for preparing a transdermal matrix system according to any one of claims 1 to 9, said method comprising the steps consisting of:
**(α)** mixing the EVA and the cellulosic material under stirring at a temperature higher than 110°C;
**(β)** incorporating into the resulting homogeneous mixture the hormone selected from the group consisting of oestrogen components, progestogen components and mixtures thereof, with the crotamiton and/or the aliphatic alcohol, if at least one of these products is required, under continued stirring at a temperature higher than 110°C, and homogenizing ;
**(γ)** incorporating into the resulting homogeneous mixture the N-substituted 2-pyrrolidone(s) of formula 1, if they are required, while stirring and at a temperature lower than 110°C, and homogenizing;
**(δ)** coating the homogeneous mixture resulting from step (β) or (γ) onto a temporary anti-adherent support, at a temperature of between 100 and 140°C, in order to obtain a deposit of 50 to 300 g/m² on said support ; and,
**(ε)** transferring the matrix thus obtained onto the final support.

12. A method for preparing a transdermal matrix system according to any one of claims 1 to 5, said method comprising the steps consisting of:
**(α)** successively incorporating while stirring the higher aliphatic alcohol, the crotamiton and/or the N-substituted 2-pyrrolidone(s) of the formula I, the hormone selected from the group consisting of oestrogen components, progestogen components and mixtures thereof, the EVA material, the cellulosic material and the chosen solvent;
**(β)** stirring the mixture thus obtained at a temperature lower than the boiling point temperature of the solvent, until homogenization of said mixture;
**(γ)** coating the homogeneous mixture resulting from step (β) onto a temporary anti-adherent support, at a temperature of between 50 and 70°C, so as to obtain a deposit of 50 to 300 g/m² on said support;
**(δ)** heating the coating thus obtained to evaporate the solvent at a temperature of between 40 and 80°C in accordance with the boiling point temperature of the latter;
**(ε)** transferring the dry matrix thus obtained to a final support.

## Patentansprüche

1. Selbstklebendes, transdermales Matrixsystem zur perkutanen Verabreichung eines Hormons mit einem Träger und einer selbstklebenden Matrix,
dadurch gekennzeichnet, daß die Matrix enthält:
(a) 40 bis 60 Gewichtsanteile eines Copolymers aus Ethylen und Vinylacetat;
(b) 5 bis 20 Gewichtsanteile eines Cellulose-Derivats;
(c) 35 bis 55 Gewichtsanteile von mindestens einer der Verbindungen ausgewählt aus:
- Crotamiton,
- N-substituierte 2-Pyrrolidone der Formel I, 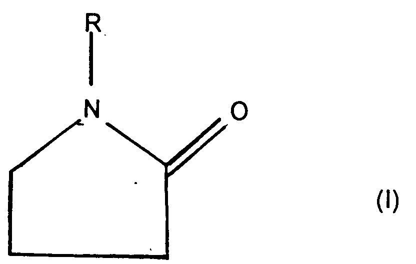 wobei der Rest R eine Alkylgruppe von C₁ bis C₁₅ Cyclohexylgruppe oder die 2-Hydroxyethylgruppe darstellt, und
- höhere aliphatische Alkohole von C₁₂ bis C₂₀, und
(d) 0,01 bis 7 Gewichtsanteile eines Hormons, ausgewählt aus der Gruppe bestehend aus den Östrogenen, den Gestagenen und ihren Mischungen.

2. System nach Anspruch 1, dadurch gekennzeichnet, daß der höhere aliphatische Alkohol 2-Octyl-1-dodecanol ist.

3. System nach Anspruch 1, dadurch gekennzeichnet, daß die Verbindung in Formel I ein N-Alkyl-2-pyrrolidon ist, wobei die Alkylgruppe eine Länge von C₁ bis C₁₅ besitzt und vorzugsweise N-Dodecyl-2-pyrrolidon ist.

4. System nach den Ansprüchen 1 bis 3, dadurch gekennzeichnet, daß das Copolymer aus Ethylen und Vinylacetat einen Gehalt an Vinylacetat zwischen 30 und 75 % seines Gewichts enthält.

5. System nach einem der Ansprüche 1 bis 4, dadurch gekennzeichnet, daß das Hormon ein Östrogen ist, vorzugsweise 17-ß-Estradiol.

6. System nach einem der Ansprüche 1 bis 4, dadurch gekennzeichnet, daß das Hormon ein Gestagen ist, vorzugsweise Norethisteronacetat.

7. System nach Anspruch 1, dadurch gekennzeichnet, daß das Hormon eine Mischung eines Östrogens und eines Gestagens ist.

8. System nach einem der Ansprüche 1 bis 7, dadurch gekennzeichnet, daß seine Matrix in einer Gesamtheit von 100 Gewichtsanteilen enthält:
(a) 46 Gewichtsanteile EVA,
(b) 10 Gewichtsanteile Ethylcellulose,
(c1) 31,5 Gewichtsanteile 2-Octyl-1-dodecanol,
(c2) 10,5 Gewichtsanteile N-Dodecyl-2-pyrrolidon und
(d) 2 Gewichtsanteile 17-ß-Estradiol.

9. System nach einem der Ansprüche 1 bis 7, dadurch gekennzeichnet, daß seine Matrix in einer Gesamtheit von 100 Gewichtsanteilen enthält:
(a) 45 Gewichtsanteile EVA
(b) 10 Gewichtsanteile Ethylcellulose
(c1) 30,75 Gewichtsanteile 2-Octyl-1-dodecanol,
(c2) 10,25 Gewichtsanteile N-Dodecyl-2-pyrrolidon und
(d) 4 Gewichtsanteile Norethisteronacetat.

10. System nach einem der Ansprüche 1 bis 7, dadurch gekennzeichnet, daß seine Matrix in einer Gesamtheit von 100 Gewichtsanteilen enthält:
(a) 42 Gewichtsanteile EVA,
(b) 15 Gewichtsanteile Ethylcellulose,
(c1) 28 Gewichtsanteile 2-Octyl-1-dodecanol,
(c2) 10 Gewichtsanteile N-Dodecyl-2-pyrrolidon,
(d1) 2 Gewichtsanteile 17-ß-Estradiol und
(d2) 3 Gewichtsanteile Norethisteronacetat.

11. Verfahren zur Herstellung eines transdermalen Matrixsystems nach einem der Ansprüche 1 bis 9, gekennzeichnet durch die folgenden Schritte:
(α) Mischen des EVAs und des Cellulose-Derivats unter Rühren und bei einer höheren Temperatur als 110 °C
(β) Zugabe des Hormons ausgewählt aus der Gruppe bestehend aus den Östrogenen, den Gestagenen und ihren Mischungen in die resultierende. homogene Mischung zusammen mit dem Crotamiton und/oder dem aliphatischen Alkohol, falls zumindest eine dieser Verbindungen beteiligt ist, unter Rühren und bei einer höheren Temperatur als 110 °C und homogenisieren,
(γ) Zugabe des oder der N-substituierten 2-Pyrrolidone der Formel 1, falls sie beteiligt sind, in die resultierende homogene Mischung unter Rühren und bei einer niedrigeren Temperatur als 110 °C und homogenisieren,
(δ) Aufstreichen der homogenen Mischung, die durch die Schritte (β) oder (γ) erhalten wurde, auf einen vorläufigen, nicht-haftenden Träger bei einer Temperatur zwischen 100 °C und 140 °C, um eine Auflage von 50 bis 300 g/m² auf dem Träger zu erhalten, und
(ε) Umsetzen der so erhaltenen Matrix auf einen endgültigen Träger.

12. Verfahren zur Herstellung eines transdermalen Matrixsystems nach einem der Ansprüche 1 bis 5, dadurch gekennzeichnet, daß es die folgenden Schritte enthält:
(α) Aufeinanderfolgende Zugabe unter Rühren des höheren aliphatischen Alkohols, des Crotamitons und/oder der N-substituierten 2-Pyrrolidone der Formel I (falls eine oder mehrere dieser Verbindungen in der Rezeptur enthalten sind), des Hormons ausgewählt aus der Gruppe bestehend aus den Östrogenen, den Gestagen und ihren Mischungen, des EVAs, des Cellulose-Derivats und des übrigen Lösungsmittels,
(β) Rühren der so erhaltenen Mischung bei einer Temperatur unterhalb des Siedepunktes des Lösungsmittels bis zu ihrer Homogenisierung,
(γ) Aufstreichen der homogenen Mischung, die durch den Schritt (β) erhalten wurde, auf einen vorläufigen, nicht-haftenden Träger bei einer Temperatur zwischen 50 °C und 70 °C, um eine Auflage von 50 bis 300 g/m² auf dem Träger zu erhalten,
(δ) Erhitzen der erhaltenen Auflage bei einer Temperatur zwischen 40 °C und 80 °C zum Verdampfen des Lösungsmittes in Abhängigkeit von dessen Siedepunkt,
(ε) Umsetzen der so erhaltenen, trockenen Matrix auf einen endgültigen Träger.
